# EUROPEAN PATENT APPLICATION

(11) **EP 4 546 489 A1**
(43) Date of publication of application: **30.04.2025**
(21) Application number: 23852488.8
(22) Date of filing: 03.08.2023
(51) Int. Cl.: H01M 10/0567, H01G 11/64, H01M 10/052

(54) **ADDITIVE FOR NON-AQUEOUS ELECTROLYTIC SOLUTION, NON-AQUEOUS ELECTROLYTIC SOLUTION, AND POWER STORAGE DEVICE**

(30) Priority: 08.08.2022 JP 2022126498
(71) Applicant: Sumitomo Seika Chemicals Co., Ltd., Kako-gun, Hyogo 675-0145 (JP)
(72) Inventor: BAN, Yuki, Kako-gun, Hyogo 675-0145 (JP); MASUHARA, Yusaku, Kako-gun, Hyogo 675-0145 (JP)
(74) Representative: Vossius & Partner Patentanwälte Rechtsanwälte mbB
(86) International application number: PCT/JP2023/028480
(87) International publication number: WO 2024/034522

(57) **Abstract**

An additive for a non-aqueous electrolytic solution includes a cyclic sulfone compound represented by Formula (1). 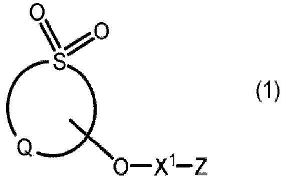 X¹ represents a sulfonyl group or carbonyl group and Z represents a monovalent group represented by Formula (21), (22), (23), or (24):

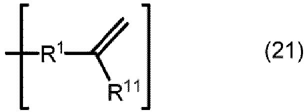

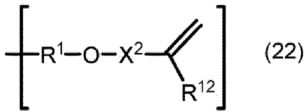

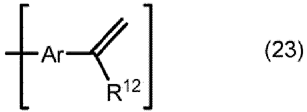

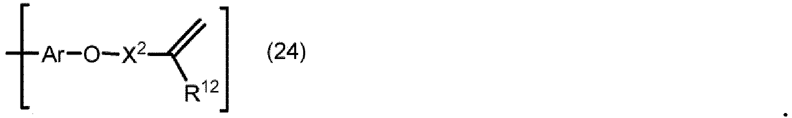

## Description

### Technical Field

The present disclosure relates to an additive for a non-aqueous electrolytic solution, a non-aqueous electrolytic solution, and a power storage device.

### Background Art

The battery capacity of non-aqueous electrolyte secondary batteries tends to decrease with repeated charging and discharging. To minimize the decrease in battery capacity due to repeated charging and discharging, various additives may be added to non-aqueous electrolytic solutions (for example, Patent Literature 1 to 6). It is known that some additives electrochemically decompose on the electrode surface during initial charging and discharging, forming a coating called a Solid Electrolyte interphace (SEI) on the electrode surface. The SEI is considered to minimize the decomposition of solvent molecules on the electrode surface, thereby minimizing the consumption of electricity associated with the decomposition of solvent molecules. That is, forming a good SEI makes it possible to contribute to minimizing the deterioration of power storage devices such as non-aqueous electrolyte secondary batteries.

### Citation List

### Patent Literature

[Patent Literature 1] Japanese Unexamined Patent Publication No. S63-102173 A
[Patent Literature 2] Japanese Unexamined Patent Publication No. H5-74486 A
[Patent Literature 3] Japanese Unexamined Patent Publication No. 2004-281368 A
[Patent Literature 4] Japanese Unexamined Patent Publication No. 2015-138597 A
[Patent Literature 5] Japanese Unexamined Patent Publication No. 2002-352852 A
[Patent Literature 6] PCT International Publication No. WO 2019/117101

### Summary of Invention

### Technical Problem

In recent years, there has been a demand for further improvement in the high-temperature characteristics of power storage devices. The present disclosure relates to maintaining a high capacity and minimizing gas generation in a power storage device even when the power storage device is exposed to a high-temperature environment.

### Solution to Problem

The present disclosure includes the following.
[1] An additive for a non-aqueous electrolytic solution including a cyclic sulfone compound represented by Formula (1): 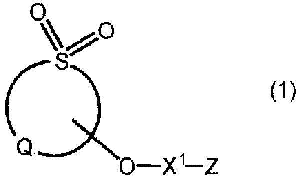 wherein,
   Q represents an alkylene group having 4 to 8 carbon atoms which may be substituted or an alkenylene group having 4 to 8 carbon atoms which may be substituted, which forms a cyclic group together with a sulfur atom of a sulfonyl group,
   X¹ represents a sulfonyl group or a carbonyl group, and
   Z represents a monovalent group represented by Formula (21), (22), (23), or (24): 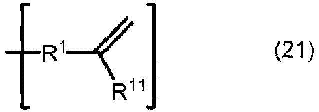 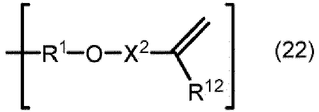 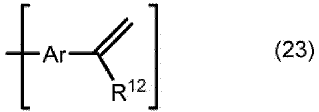 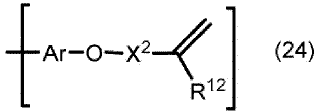 wherein,
      R¹ represents a direct bond or an alkylene group having 1 to 6 carbon atoms which may be substituted,
      R¹¹ and R¹² represent a hydrogen atom, a methyl group, or an ethyl group,
      when Q is a tetramethylene group and X¹ is a sulfonyl group,
      R¹¹ is a methyl group or an ethyl group,
      X² represents a sulfonyl group or a carbonyl group, and
      Ar represents an arylene group which may be substituted.
[1'] Use of the cyclic sulfone compound represented by Formula (1) for adding to a non-aqueous electrolytic solution.
[2] The additive for a non-aqueous electrolytic solution according to [1], in which X¹ is a sulfonyl group.
[3] The additive for a non-aqueous electrolytic solution according to [1] or [2], in which the cyclic sulfone compound is a compound represented by Formula (11): 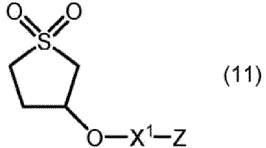 wherein X¹ and Z in Formula (11) are identical to X¹ and Z in Formula (1).
[4] The additive for a non-aqueous electrolytic solution according to [1] or [2], in which the cyclic sulfone compound is a compound represented by Formula (12): 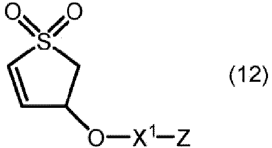 wherein X¹ and Z in Formula (12) are identical to X¹ and Z in Formula (1).
[5] The additive for a non-aqueous electrolytic solution according to any of [1] to [4], in which
   R¹ is a direct bond or an alkylene group having 1 to 3 carbon atoms which may be substituted with a fluorine atom, and
   Ar is an arylene group which may be substituted with a fluorine atom or a fluoroalkyl group.
[6] A non-aqueous electrolytic solution including the additive for a non-aqueous electrolytic solution according to any of [1] to [5], a non-aqueous solvent, and an electrolyte.
[7] A power storage device including the non-aqueous electrolytic solution according to [6], a positive electrode, and a negative electrode.
[8] A lithium-ion battery including the non-aqueous electrolytic solution according to [6], a positive electrode, and a negative electrode.
[9] A lithium-ion capacitor including the non-aqueous electrolytic solution according to [6], a positive electrode, and a negative electrode.
[10] A cyclic sulfone compound represented by Formula (11S) or (12S): 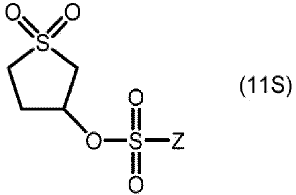 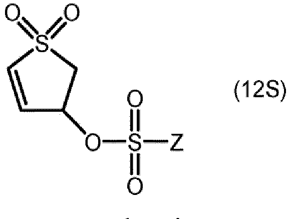 wherein Z represents a monovalent group represented by Formula (21A) or (22A): 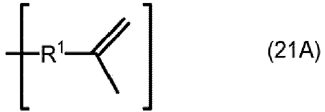 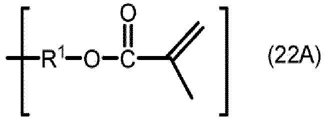 wherein R¹ represents an alkylene group having 1 to 6 carbon atoms which may be substituted with a fluorine atom.
[11] The cyclic sulfone compound according to [10], the following formula: 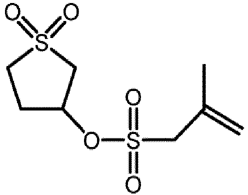
[12] The cyclic sulfone compound according to [10], represented by the following formula: 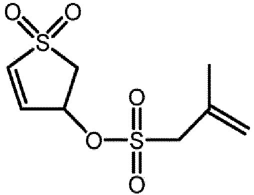
[13] The cyclic sulfone compound according to [10], the following formula:

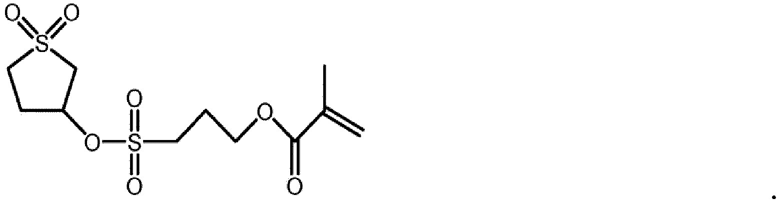

### Advantageous Effects of Invention

It is possible to maintain a high capacity and minimize gas generation in a power storage device even when the power storage device is exposed to a high-temperature environment. The additive for a non-aqueous electrolytic solution according to the present disclosure may also have better performance in terms of stability and handling.

### Brief Description of Drawings

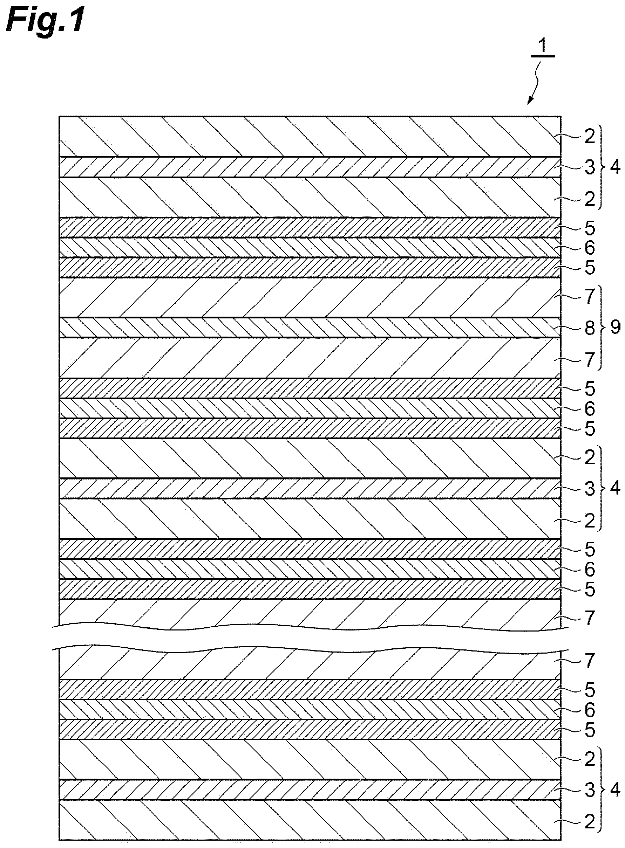

Fig. 1 is a cross-sectional view showing an example of a power storage device.

### Description of Embodiments

The present invention is not limited to the following examples.

An example of an additive for a non-aqueous electrolytic solution includes one or more cyclic sulfone compounds represented by Formula (1).

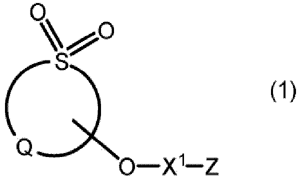

In Formula (1), Q is a divalent group forming a cyclic group together with the sulfur atom of the sulfonyl group and represents an alkylene group having 4 to 8 carbon atoms which may be substituted or an alkenylene group having 4 to 8 carbon atoms which may be substituted. X¹ represents a sulfonyl group or a carbonyl group. Z is a monovalent group represented by Formula (21), (22), (23), or (24).

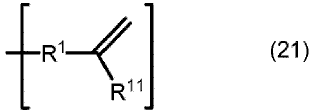

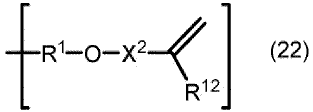

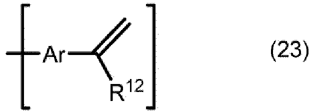

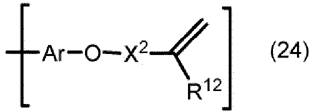

R¹ in Formulas (21) and (22) is a direct bond or an alkylene group having 1 to 6 carbon atoms which may be substituted. In Formulas (21) to (24), R¹¹ and R¹² are hydrogen atoms, methyl groups, or ethyl groups. However, when Q is a tetramethylene group and X¹ is a sulfonyl group, R¹¹ is a methyl group or an ethyl group. X² in Formulas (22) and (24) represents a sulfonyl group or a carbonyl group. Ar in Formulas (23) and (24) represents an arylene group which may be substituted.

In the present specification, "which may be substituted" means that one or more of the hydrogen atoms of each group may be substituted with a substituent.

It is considered that, when the cyclic sulfone compound represented by Formula (1) undergoes electrochemical reduction during initial charging and discharging, the terminal carbon-carbon unsaturated bonds are polymerized on the electrode, thereby multiplying to form a strong SEI. In addition, the formed SEI is considered to contain a large number or a high density of polar groups including O, S, and the like. In this manner, a stable SEI including a high density of polar groups is considered to be able to minimize the reaction between the electrode active material and the solvent or the like, which is a side reaction, and is considered to contribute to, for example, improving the capacity retention rate during high-temperature storage and minimizing gas generation.

Q in Formula (1) is a divalent group forming a cyclic sulfone and is substituted at any position with a group represented by -O-X¹-Z. Q represents an alkylene group having 4 to 8 carbon atoms which may be substituted or an alkenylene group having 4 to 8 carbon atoms which may be substituted, and may be further substituted with a substituent other than -O-X¹-Z.

The alkylene group having 4 to 8 carbon atoms as Q in Formula (1) may be a linear alkylene group or a branched alkylene group. Q may be an alkylene group having 1 to 6 carbon atoms which may be substituted with a halogen atom or an alkylene group having 1 to 6 carbon atoms which may be substituted with a fluorine atom.

When Q is an alkylene group having 4 to 8 carbon atoms, examples thereof include a tetramethylene group, a pentamethylene group, a 1-methyltetramethylene group, a 2-methyltetramethylene group, a hexamethylene group, a 1-methylpentamethylene group, a 2-methylpentamethylene group, a 3-methylpentamethylene group, a 1-ethyltetramethylene group, a 1,2-dimethyltetramethylene group, a heptamethylene group, a 1-ethylpentamethylene group, a 1,2-dimethylpentamethylene group, and an octamethylene group.

When Q is an alkylene group having 4 to 8 carbon atoms substituted with a fluorine atom, examples thereof include a 1-fluorotetramethylene group, a 2,3-difluorotetramethylene group, a 1-fluoropentamethylene group, a 2-(trifluoromethyl)tetramethylene group, a 1-fluoro-2-(trifluoromethyl)tetramethylene group, a 1-fluorohexamethylene group, a 3-(trifluoromethyl)pentamethylene group, a 1,2-bis(trifluoromethyl)tetramethylene group, a 2-(pentafluoroethyl)tetramethylene group, a 1-fluoroheptamethylene group, a 1-(trifluoromethyl)hexamethylene group, a 1-(hexafluoroethyl)pentamethylene group, a 1- fluorooctamethylene group, and a 1,8-difluorooctamethylene group.

The alkenylene group having 4 to 8 carbon atoms as Q in Formula (1) may be a linear alkenylene group or a branched alkenylene group. Q may be an alkenylene group having 4 to 8 carbon atoms which may be substituted with a halogen atom or an alkenylene group having 4 to 8 carbon atoms which may be substituted with a fluorine atom.

When Q is an alkenylene group having 4 to 8 carbon atoms, examples thereof include -CH=CHCH₂CH₂-, -CH₂CH=CHCH₂-, - CH=CHCH₂CH₂CH₂-, -CH₂CH=CHCH₂CH₂-, -CH=C(CH₃)CH₂CH₂-, - CH₂(CH₃)CH=CHCH₂-, -CH=CHCH₂CH₂CH₂CH₂-, - CH₂CH=CHCH₂CH₂CH₂-, -CH₂CH₂CH=CHCH₂CH₂-, - C(CH₃)=CHCH₂CH₂CH₂-, -CH=C(CH₃)CH₂CH₂CH₂-, - CH(CH₃)CH=CHCH₂CH₂-, -CH=CHCH₂CH₂CH₂CH₂CH₂-, - C(CH₃)=CHCH₂CH₂CH₂CH₂-, -C(CH₂CH₃)=CHCH₂CH₂CH₂, - C(CH₃)=C(CH₃)CH₂CH₂CH₂-, and -CH=CHCH₂CH₂CH₂CH₂CH₂CH₂-.

When Q is a linear or branched alkenylene group having 4 to 8 carbon atoms substituted with a fluorine atom, examples thereof include -CH=CHCF₂CH₂-, -CH₂CH=CH₂CF₂-, -CF=CHCH₂CH₂-, - CH=CFCH₂CH₂-, -CH=CHCHFCH₂CH₂-, -CF=CHCH₂CH₂CH₂-, - CH=CH-CH(CF₃)CH₂-, -CF=CHCH₂CH₂CH₂CH₂-, - CH=CHCH(CF)CH₂CH₂-, -CF=CHCH₂CH₂CH₂CH₂CH₂-, and - CF=CHCH₂CH₂CH₂CH₂CH₂CH₂-.

From the viewpoint of further improving the capacity retention rate during high-temperature storage, Q may form a five-membered ring together with the sulfur atom of the sulfonyl group. In this case, for example, Q may be an alkylene group having 4 carbon atoms which may be substituted with a halogen atom or an alkenylene group having 4 carbon atoms which may be substituted with a halogen atom. From the same viewpoint, Q may be an alkylene group having 4 carbon atoms which may be substituted with a fluorine atom or an alkenylene group having 4 carbon atoms which may be substituted with a fluorine atom.

X¹ in Formula (1) may be a sulfonyl group from the viewpoint of further minimizing gas generation.

R¹ in Formulas (21) and (22) is an unsubstituted or substituted alkylene group having 1 to 6 carbon atoms. The alkylene group having 1 to 6 carbon atoms may be a linear alkylene group or a branched alkylene group. R¹ may be 1 to 6 carbon atoms which may be substituted with a halogen atom, or may be an alkylene group having 1 to 3 carbon atoms.

In the present specification, "may be substituted with a halogen atom" means that one or more of the hydrogen atoms of each group may be substituted with a halogen atom. The halogen atom as a substituent may be an iodine atom, a bromine atom, a chlorine atom, or a fluorine atom and may be a fluorine atom from the viewpoint of further reducing the battery resistance.

When R¹ is an unsubstituted alkylene group having 1 to 6 carbon atoms, examples thereof include a methylene group, a dimethylene group (ethylene group), a trimethylene (propane-1,3-diyl group), a propylene group (propane-1,2-diyl group), a tetramethylene group (butane-1,3-diyl group), a 1-methyltrimethylene group, a 2-methyltrimethylene group, a 1,1-dimethyldimethylene group, a 1,2-dimethyldimethylene group, an ethyldimethylene group, a pentamethylene group (pentane-1,5-diyl group), and a hexamethylene group (hexane-1,6-diyl group).

When R¹ is an alkylene group having 1 to 6 carbon atoms substituted with a fluorine atom, examples of alkylene groups include a fluoromethylene group, a difluoromethylene group, a 1-fluorodimethylene group, a 1,1-difluorodimethylene group, a 1,2-difluorodimethylene group, a 1,1,2-trifluorodimethylene group, a 1,1,2,2-tetrafluorodimethylene group, a trifluoromethylmethylene group, a 1-fluorotrimethylene group, a 2-fluorotrimethylene group, a 1,1-difluorotrimethylene group, a 1,2-difluorotrimethylene group, a 1,3-difluorotrimethylene group, a 2,2-difluorotrimethylene group, a 1,2,3-trifluorotrimethylene group, a 1,1,2,2,3,3-hexafluorotrimethylene group, a trifluoromethyldimethylene group, a 1-fluorotetramethylene group, a 2-fluorotetramethylene group, a 1,1-difluorotetramethylene group, a 1,1,2,2-tetrafluorotetramethylene group, a 1,1,2,2,3,3-hexafluorotetramethylene group, a 1,1,2,2,3,3,4,4-octafluorotetramethylene group, a 1-(trifluoromethyl)trimethylene group, a 2-(trifluoromethyl)trimethylene group, a 1-fluoropentamethylene group, a 2-fluoropentamethylene group, a 3-fluoropentamethylene group, a 1,1-difluoropentamethylene group, a 1,1,2,2-tetrafluoropentamethylene group, a 1,1,2,2,3,3-hexafluoropentamethylene group, a 1,1,2,2,3,3,4,4-octafluoropentamethylene group, a 1,1,2,2,3,3,4,4,5,5-decafluoropentamethylene group, a 1-(trifluoromethyl)tetramethylene group, a 2-(trifluoromethyl)tetramethylene group, a 1,2-bis(trifluoromethyl)trimethylene group, a 2,2-bis(trifluoromethyl)trimethylene group, a 1-(pentafluoroethyl)trimethylene group, a 2-(pentafluoroethyl)trimethylene group, a 1-fluorohexamethylene group, a 2-fluorohexamethylene group, a 3-fluorohexamethylene group, a 1,1-difluorohexamethylene group, a 1,1,2,2-tetrafluorohexamethylene group, a 1,1,2,2,3,3-hexafluorohexamethylene group, a 1,1,2,2,3,3,4,4-octafluorohexamethylene group, a 1,1,2,2,3,3,4,4,5,5-decafluorohexamethylene group, a 1,1,2,2,3,3,4,4,5,5,6,6-dodecafluorohexamethylene group, a 1-(trifluoromethyl)pentamethylene group, a 2-(trifluoromethyl)pentamethylene group, a 3-(trifluoromethyl)pentamethylene group, a 1,1-di(trifluoromethyl)tetramethylene group, a 1,2-di(trifluoromethyl)tetramethylene group, a 1,3-di(trifluoromethyl)tetramethylene group, a 1,4-di(trifluoromethyl)tetramethylene group, a 2,2-di(trifluoromethyl)tetramethylene group, a 2,3-di(trifluoromethyl)tetramethylene group, a 1-(pentafluoroethyl)tetramethylene group, a 2-(pentafluoroethyl)tetramethylene group, a 1,2,3-tri(trifluoromethyl)trimethylene group, and a 1-(heptafluoropropyl)trimethylene group.

R¹ may be a methylene group which may be substituted with a fluorine atom, a dimethylene group which may be substituted with a fluorine atom, or a trimethylene group which may be substituted with a fluorine atom.

It is possible for Ar in Formulas (23) and (24) to be an unsubstituted arylene group or a substituted arylene group. Ar may be an arylene group which may be substituted with a halogen atom, a halogenated alkyl group, an alkyl group (for example, a methyl group), or a combination thereof. Ar may be an arylene group substituted with a fluorine atom or a fluoroalkyl group (for example, a fluoromethyl group) or may be an unsubstituted arylene group. The arylene group substituted with a fluorine atom or a fluoroalkyl group may be further substituted with an alkyl group. The number of carbon atoms in the arylene group is 6 to 10 and may be 6.

When Ar is an unsubstituted arylene group, an example thereof is a benzene-1,4-diyl group.

When Ar is an arylene group substituted with a fluorine atom or a fluoroalkyl group, examples thereof include a 2-fluoroarylene group, a 2,3-difluoroarylene group, a 2,5-difluoroarylene group, a 2,6-difluoroarylene group, a 2,3,5-trifluoroarylene group, a 2,3,6-trifluoroarylene group, a 2,3,5,6-tetrafluoroarylene group, a 2-methyl-3-fluoroarylene group, a 2-methyl-5-fluoroarylene group, a 2-methyl-6-fluoroarylene group, and a 2-trifluoromethylarylene group.

When Ar is an arylene group substituted with an alkyl group, examples thereof include a 2,3-dimethylarylene group, a 2,3,5-trimethylarylene group, and a 2,3,5,6-tetramethylarylene group.

The cyclic sulfone compound represented by Formula (1) may be a compound represented by Formula (11) or a compound represented by Formula (12). X¹ and Z in Formulas (11) and (12) are the same groups as X¹ and Z in Formula (1), including examples thereof. These compounds are advantageous in terms of the ease of obtaining raw materials and the ease of synthesis.

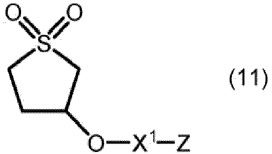

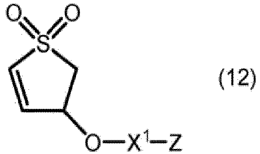

In particular, the cyclic sulfone compound may be a compound represented by Formula (11S) or a compound represented by Formula (12S). 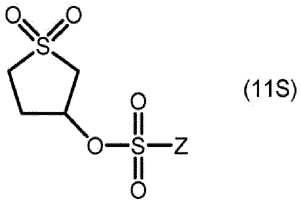 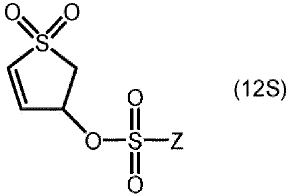 Z in Formulas (11S) and (12S) represents a monovalent group represented by Formula (21A) or (22A).

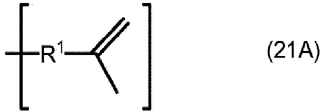

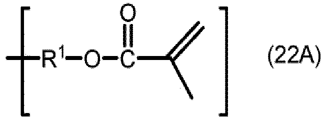

In Formulas (21A) and (22A), R¹ represents an alkylene group having 1 to 6 carbon atoms which may be substituted with a fluorine atom and Ar represents an arylene group which may be substituted with a fluorine atom or a fluoroalkyl group.

Specific examples of the cyclic sulfone compound represented by Formula (1) include the compounds represented by Formulas (1-1) to (1-18) shown in Table 1 and Table 2 below.

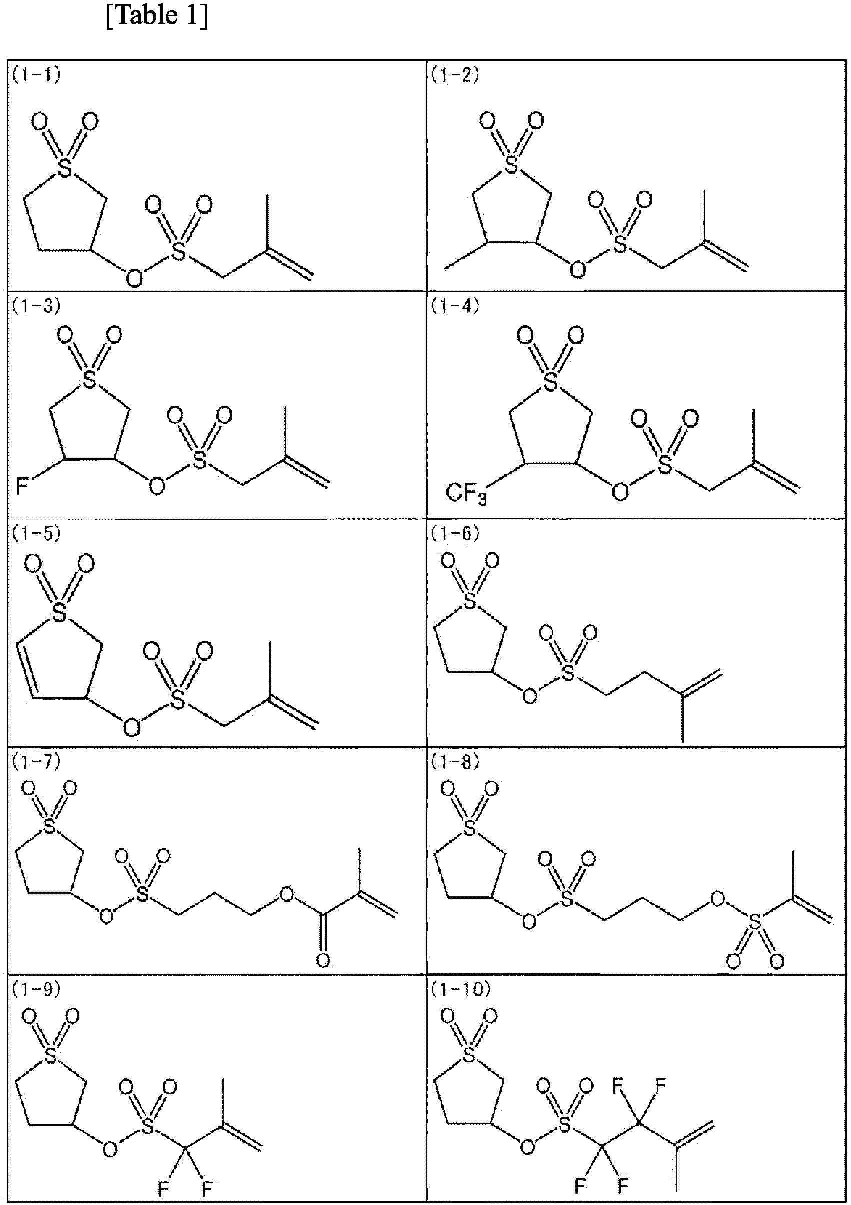

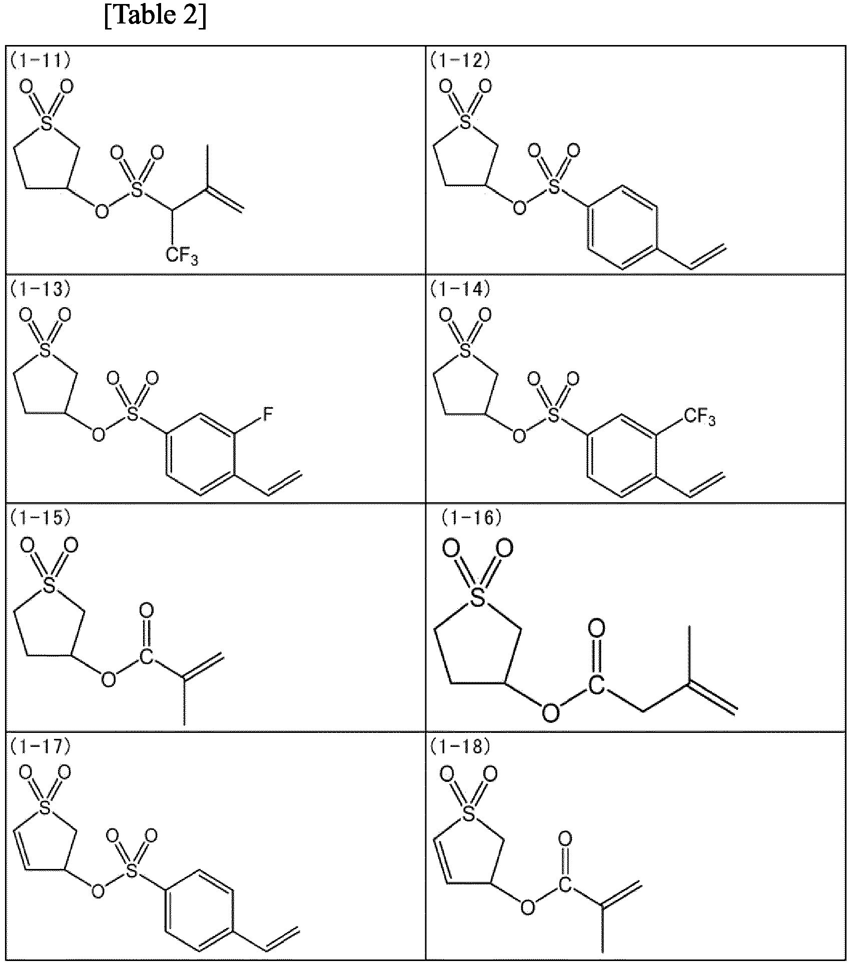

It is possible to synthesize the cyclic sulfone compound represented by Formula (1) by a usual method understood by a person skilled in the art. For example, it is possible to synthesize the cyclic sulfone compound represented by Formula (1) by a method including reacting hydroxysulfolane having a group corresponding to the cyclic sulfone compound with an acid chloride compound corresponding to the cyclic sulfone compound in a solvent in the presence of a base such as triethylamine.

The additive for a non-aqueous electrolytic solution according to the present disclosure may include, in addition to the cyclic sulfone compound represented by Formula (1), other components different from the cyclic sulfone compound as necessary. Examples of other components include negative electrode protectors, positive electrode protectors, flame retardants, overcharge inhibitors, cyclic carbonate compounds, nitrile compounds, isocyanate compounds, compounds having an acetylene-1,2-diyl group (-C≡C-), compounds having a sulfonyl group (>S(=O)₂) different from the cyclic sulfone compound of Formula (1), phosphate ester compounds, acid anhydrides, cyclic phosphazene compounds, boroxine derivatives, compounds including silicon atoms, and alkyl metal salt compounds (for example, lithium salt compounds). These other components may be included in the non-aqueous electrolytic solution together with the cyclic sulfone compound represented by Formula (1) as additives for improving the characteristics of a power storage device. It is also possible for the alkali metal salt compound to function as an electrolyte in the non-aqueous electrolytic solution.

Examples of cyclic carbonate compounds include 4-fluoro-1,3-dioxolane-2-one (FEC), trans- or cis-4,5-difluoro-1,3-dioxolane-2-one (DFEC), vinylene carbonate (VC), vinyl ethylene carbonate (VEC), and 4-ethynyl-1,3-dioxolane-2-one (EEC). The cyclic carbonate compound may be VC, FEC, VEC, or a combination thereof.

Examples of nitrile compounds include acetonitrile, propionitrile, succinonitrile, glutaronitrile, adiponitrile, pimelonitrile, suberonitrile, and sebaconitrile. The nitrile compound may be succinonitrile, adiponitrile, or a combination thereof.

Examples of isocyanate compounds include methyl isocyanate, ethyl isocyanate, butyl isocyanate, phenyl isocyanate, tetramethylene diisocyanate, hexamethylene diisocyanate, octamethylene diisocyanate, 1,4-phenylene diisocyanate, 2-isocyanatoethyl acrylate, and 2-isocyanatoethyl methacrylate.

Examples of compounds having an acetylene-1,2-diyl group (-C≡C-) include 2-propynyl methyl carbonate, 2-propynyl acetate, 2-propynyl formate, 2-propynyl methacrylate, 2-propynyl methanesulfonate, 2-propynyl vinylsulfonate, 2-propynyl 2-(methanesulfonyloxy)propionate, di(2-propynyl)oxalate, methyl-2-propynyl oxalate, ethyl-2-propynyl oxalate, di(2-propynyl) glutarate, 2-butyne-1,4-diyl dimethanesulfonate, 2-butyne-1,4-diyl diformate, and 2,4-hexadiyne-1,6-diyl dimethanesulfonate.

Examples of compounds having a sulfonyl group (>S(=O)₂) include sultones such as 1,3-propane sultone (PS), 1,3-butane sultone, 2,4-butane sultone, 1,4-butane sultone, 1,3-propene sultone, 2,2-dioxide-1,2-oxathiolan-4-yl acetate, and 5,5-dimethyl-1,2-oxathiolan-4-one 2,2-dioxide, cyclic sulfites such as ethylene sulfite, ethylene sulfate, hexahydrobenzo[1,3,2]dioxathiolan-2-oxide (also called 1,2-cyclohexanediol cyclic sulfite), and 5-vinyl-hexahydro-1,3,2-benzodioxathiol-2-oxide, sulfonic acid esters such as butane-2,3-diyldimethanesulfonate, butane-1,4-diyldimethanesulfonate, methylenemethanedisulfonate, and 1,3-propanedisulfonic anhydride, divinyl sulfone, 1,2-bis(vinylsulfonyl)ethane, and bis(2-vinylsulfonylethyl)ether.

Examples of phosphate ester compounds include trimethyl phosphate, tributyl phosphate, trioctyl phosphate, tris(2,2,2-trifluoroethyl)phosphate, bis(2,2,2-trifluoroethyl)methyl phosphate, bis(2,2,2-trifluoroethyl)ethyl phosphate, bis(2,2,2-trifluoroethyl)2,2-difluoroethyl phosphate, bis(2,2,2-trifluoroethyl)2,2,3,3-tetrafluoropropyl phosphate, bis(2,2-difluoroethyl)2,2,2-trifluoroethyl phosphate, bis(2,2,3,3-tetrafluoropropyl)2,2,2-trifluoroethyl phosphate, (2,2,2-trifluoroethyl)(2,2,3,3-tetrafluoropropyl)methyl phosphate, tris(1,1,1,3,3,3-hexafluoropropane-2-yl) phosphate, methyl methylene bisphosphonate, ethyl methylene bisphosphonate, methyl ethylene bisphosphonate, ethyl ethylene bisphosphonate, methyl butylene bisphosphonate, ethyl butylene bisphosphonate, methyl 2-(dimethyl phosphoryl) acetate, ethyl 2-(dimethyl phosphoryl) acetate, methyl 2-(diethyl phosphoryl) acetate, ethyl 2-(diethyl phosphoryl) acetate, 2-propynyl 2-(dimethyl phosphoryl) acetate, 2-propynyl 2-(diethyl phosphoryl) acetate, methyl 2-(dimethoxy phosphoryl) acetate, ethyl 2-(dimethoxy phosphoryl) acetate, methyl 2-(diethoxy phosphoryl) acetate, ethyl 2-(diethoxy phosphoryl) acetate, 2-propynyl 2-(dimethoxy phosphoryl) acetate, 2-propynyl 2-(diethoxy phosphoryl) acetate, methyl pyrophosphate, and ethyl pyrophosphate.

Examples of acid anhydrides include acetic anhydride, propionic anhydride, succinic anhydride, maleic anhydride, 3-allyl succinic anhydride, glutaric anhydride, itaconic anhydride, and 3-sulfo-propionic anhydride.

Examples of cyclic phosphazene compounds include methoxypentafluorocyclotriphosphazene, ethoxypentafluorocyclotriphosphazene, phenoxypentafluorocyclotriphosphazene, and ethoxyheptafluorocyclotetraphosphazene.

Examples of compounds including boron atoms include boroxine, trimethylboroxine, trimethoxyboroxine, triethylboroxine, triethoxyboroxine, triisopropylboroxine, triisopropoxyboroxine, tri-n-propylboroxine, tri-n-propoxyboroxine, tri-n-butylboroxine, tri-n-butyloxyboroxine, triphenylboroxine, triphenoxyboroxine, tricyclohexylboroxine, and tricyclohexoxyboroxine.

Examples of compounds including silicon atoms include hexamethylcyclotrisiloxane, hexaethylcyclotrisiloxane, hexaphenylcyclotrisiloxane, 1,3,5-trimethyl-1,3,5-trivinylcyclotrisiloxane, octamethylcyclotetrasiloxane, decamethylcyclopentasiloxane, trimethylfluorosilane, triethylfluorosilane, tripropylfluorosilane, phenyldimethylfluorosilane, triphenylfluorosilane, vinyldimethylfluorosilane, vinyldiethylfluorosilane, vinyldiphenylfluorosilane, divinyldifluorosilane, divinyldimethylsilane, trimethoxyfluorosilane, triethoxyfluorosilane, dimethyldifluorosilane, diethyldifluorosilane, trivinylfluorosilane, trivinylmethylsilane, divinyldifluorosilane, ethylvinyldifluorosilane, methyltrifluorosilane, ethyltrifluorosilane, hexamethyldisiloxane, 1,3-diethyltetramethyldisiloxane, hexaethyldisiloxane, octamethyltrisiloxane, methoxytrimethylsilane, ethoxytrimethylsilane, dimethoxydimethylsilane, trimethoxymethylsilane, tetramethoxysilane, tetravinylsilane, tetraallylsilane, tetrabutenylsilane, bis(trimethylsilyl)peroxide, trimethylsilyl acetate, triethylsilyl acetate, trimethylsilyl propionate, trimethylsilyl methacrylate, trimethylsilyl trifluoroacetate, trimethylsilyl methanesulfonate, trimethylsilyl ethanesulfonate, triethylsilyl methanesulfonate, trimethylsilyl fluoromethanesulfonate, bis(trimethylsilyl)sulfate, tris(trimethylsiloxy)boron, tris(trimethylsilyl)phosphate, and tris(trimethylsilyl)phosphite.

Examples of lithium salt compounds include lithium salts having a phosphate skeleton such as lithium difluorophosphate, lithium bisoxalatoborate (LiBOB), lithium tetrafluoro(oxalato)phosphate (LiTFOP), lithium difluorooxalatoborate (LiDFOB), lithium difluorobisoxalatophosphate (LiDFOP), lithium tetrafluoroborate, lithium bisfluorosulfonylimide, lithium tetrafluoro(oxalato)phosphate, and Li₂PO₃F, as well as lithium salts having an S(=O) group such as lithium trifluoro((methanesulfonyl)oxy)borate, lithium pentafluoro((methanesulfonyl)oxy)phosphate, lithium methylsulfate, lithium ethylsulfate, lithium 2,2,2-trifluoroethylsulfate, and lithium fluorosulfonate.

Other examples of alkali metal salt compounds include sodium difluorophosphate, potassium difluorophosphate, sodium bisoxalatoborate, potassium bisoxalatoborate, sodium tetrafluoro(oxalato)phosphate, potassium tetrafluoro(oxalato)phosphate, sodium difluorobis(oxalato)phosphate, potassium difluorobis(oxalato)phosphate, sodium difluorooxalatoborate, and potassium difluorooxalatoborate.

One example of a non-aqueous electrolytic solution contains an additive including a cyclic sulfone compound represented by Formula (1), a non-aqueous solvent, and an electrolyte.

It is possible to appropriately determine the content of the cyclic sulfone compound represented by Formula (1) in the non-aqueous electrolytic solution by taking into account the effect of the additive and the content may be, for example, in a range of 0.001 to 10% by mass based on the total mass of the non-aqueous electrolytic solution. When the content of the cyclic sulfone compound represented by Formula (1) is 0.001% by mass or more and 10% by mass or less based on the total mass of the non-aqueous electrolytic solution, a coating (SEI) of an appropriate thickness is formed on the electrode, which tends to make it possible to particularly effectively minimize the increase in resistance. From the same viewpoint, the content of the cyclic sulfone compound represented by Formula (1) may be 0.05% by mass or more and 8% by mass or less, 0.05% by mass or more and 5% by mass or less, 0.05% by mass or more and 4% by mass or less, 0.05% by mass or more and 3% by mass or less, 0.1% by mass or more and 8% by mass or less, 0.1% by mass or more and 5% by mass or less, 0.1% by mass or more and 4% by mass or less, or 0.1% by mass or more and 3% by mass or less based on the total mass of the non-aqueous electrolytic solution.

When the non-aqueous electrolytic solution further includes, as an additive, another compound different from the cyclic sulfone compound represented by Formula (1), the content of the other compound may be 0.001% by mass or more, 10% by mass or less, or 0.001 to 10% by mass based on the total mass of the non-aqueous electrolytic solution.

The non-aqueous solvent may be an aprotic solvent from the viewpoint of keeping the viscosity of the non-aqueous electrolytic solution low. The aprotic solvent may include at least one selected from the group consisting of cyclic carbonates, chain carbonates, aliphatic carboxylic acid esters, lactones, lactams, cyclic ethers, chain ethers, sulfones, nitriles, and halogen derivatives thereof. The aprotic solvent may include a cyclic carbonate or a chain carbonate or may include a combination of a cyclic carbonate and a chain carbonate.

Examples of cyclic carbonates include ethylene carbonate, propylene carbonate, butylene carbonate, and fluoroethylene carbonate. Examples of chain carbonates include dimethyl carbonate, diethyl carbonate, and ethyl methyl carbonate. Examples of aliphatic carboxylic acid esters include methyl acetate, ethyl acetate, methyl propionate, ethyl propionate, methyl butyrate, methyl isobutyrate, and methyl trimethylacetate. Examples of lactones include γ-butyrolactone. Examples of lactams include ε-caprolactam and N-methylpyrrolidone. Examples of cyclic ethers include tetrahydrofuran, 2-methyltetrahydrofuran, tetrahydropyran, and 1,3-dioxolane. Examples of chain ethers include 1,2-diethoxyethane and ethoxymethoxyethane. Examples of sulfones include sulfolane. Examples of nitriles include acetonitrile. Examples of halogen derivatives include halogen derivatives of cyclic carbonates such as 4-fluoro-1,3-dioxolane-2-one, 4-chloro-1,3-dioxolane-2-one, and 4,5-difluoro-1,3-dioxolane-2-one. The above may be used alone or in a combination of two or more. These non-aqueous solvents are particularly suitable for use in secondary batteries such as lithium-ion batteries.

The content of the non-aqueous solvent in the non-aqueous electrolytic solution may be, for example, 70 to 99% by mass based on the total mass of the non-aqueous electrolytic solution.

The electrolyte may be a lithium salt that is an ion source for lithium ions or a sodium salt that is an ion source for sodium ions. The electrolyte may include at least one selected from the group consisting of LiAlCl₄, LiBF₄, LiPF₆, LiClO₄, LiTFSI (lithium bistrifluoromethanesulfonimide), LiFSI (lithium bisfluorosulfonimide), LiAsF₆, and LiSbF₆. Examples of sodium salts include NaPF₆, NaBF₄, NaClO₄, NaAsF, NaCF₃SO₃, NaN(CF₃SO₂)₂, NaN(C₂F₅SO₂)₂, and NaC(CF₃SO₂)3. The electrolytic solution may include LiBF₄, LiPF₆, or a combination thereof, from viewpoints such as that the degree of dissociation is high, it is possible to increase the ionic conductivity of the electrolyte, and the oxidation-reduction resistance properties have the effect of minimizing the performance deterioration of the power storage device due to long-term use.

When the electrolyte is LiBF₄, LiPF₆, or a combination thereof, the non-aqueous solvent may include a cyclic carbonate and a chain carbonate. LiBF₄ and/or LiPF₆ may be combined with ethylene carbonate and diethyl carbonate.

The concentration of the electrolyte in the non-aqueous electrolytic solution may be 0.1 mol/L or more and 2.0 mol/L or less based on the total volume of the non-aqueous electrolytic solution. When the concentration of the electrolyte based on the total volume of the non-aqueous electrolytic solution is 0.1 mol/L or more, good conductivity of the non-aqueous electrolytic solution and the like are easily obtained. When the concentration of the electrolyte based on the total volume of the non-aqueous electrolytic solution is 2.0 mol/L or less, it is possible to minimize the viscosity increase of the non-aqueous electrolytic solution and to particularly easily ensure the mobility of ions. From the same viewpoint, the concentration of the electrolyte may be 0.5 mol/L or more and 1.5 mol/L or less based on the total volume of the non-aqueous electrolytic solution.

The non-aqueous electrolytic solution may include one or more lithium salts (first lithium salt) selected from the group consisting of LiAlCl₄, LiBF₄, LiPF₆, LiClO₄, LiTFSI (lithium bistrifluoromethanesulfonimide), LiFSI (lithium bisfluorosulfonimide), LiAsF₆, and LiSbF₆, and one or more second lithium salts different from the first lithium salt. Examples of the second lithium salts include lithium salts having a phosphate skeleton such as lithium difluorophosphate, lithium bisoxalatoborate (LiBOB), lithium tetrafluoro(oxalato)phosphate (LiTFOP), lithium difluorooxalatoborate (LiDFOB), lithium difluorobisoxalatophosphate (LiDFOP), lithium tetrafluoroborate, lithium bisfluorosulfonylimide, lithium tetrafluoro(oxalato)phosphate, and Li₂PO₃F, as well as lithium salts having an S(=O) group such as lithium trifluoro((methanesulfonyl)oxy)borate, lithium pentafluoro((methanesulfonyl)oxy)phosphate, lithium methylsulfate, lithium ethylsulfate, lithium 2,2,2-trifluoroethylsulfate, and lithium fluorosulfonate. The second lithium salt may include one or more lithium salts selected from the group consisting of lithium difluorophosphate, lithium bisoxalatoborate, lithium tetrafluoro(oxalato)phosphate, lithium difluorooxalatoborate, lithium difluorobisoxalatophosphate, lithium methylsulfate, lithium ethylsulfate, and lithium fluorosulfonate.

The concentration of the second lithium salt in the non-aqueous electrolytic solution may be 1.0 mol/L or less based on the volume of the non-aqueous electrolytic solution. When the concentration of the second lithium salt is 1.0 mol/L or less, the viscosity of the non-aqueous electrolytic solution is unlikely to increase, thus, it is possible to sufficiently ensure the mobility of ions. From the same viewpoint, the concentration of the second lithium salt may be 0.8 mol/L or less or 0.5 mol/L or less.

The non-aqueous electrolytic solution is prepared, for example, by dissolving an additive for a non-aqueous electrolytic solution including a compound represented by Formula (1) in a non-aqueous solvent together with an electrolyte and other components added as necessary.

The power storage device is mainly formed of the above-mentioned non-aqueous electrolytic solution, a positive electrode, and a negative electrode. Specific examples of power storage devices include non-aqueous electrolyte secondary batteries (lithium-ion batteries, sodium ion batteries, and lithium sulfur batteries) and electric double layer capacitors (lithium-ion capacitors and sodium ion capacitors). The non-aqueous electrolytic solution according to the present disclosure is particularly useful in uses as lithium-ion batteries and lithium-ion capacitors.

Fig. 1 is a cross-sectional view schematically showing an example of a non-aqueous electrolyte secondary battery (for example, a lithium-ion battery) that is a power storage device. A non-aqueous electrolyte secondary battery 1 shown in Fig. 1 is provided with negative electrodes 4 and positive electrodes 9 that are alternately stacked, non-aqueous electrolytic solutions 5 arranged between the negative electrodes 4 and the positive electrodes 9, and separators 6 provided in the non-aqueous electrolytic solutions 5. Here, a plurality of the negative electrode 4 and the positive electrode 9 are stacked such that the main surface of the negative electrode 4 faces the main surface of the positive electrode 9 via the separator 6. The non-aqueous electrolyte secondary battery 1 is provided with seven layers of the negative electrodes 4 and six layers of the positive electrodes 9, but some of the repeated structures are not shown in Fig. 1. The negative electrodes 4 have a negative electrode current collector 3 and negative electrode active material layers 2 provided on both sides of the negative electrode current collector 3. The positive electrodes 9 have a positive electrode current collector 8 and positive electrode active material layers 7 provided on both sides of the positive electrode current collector 8. The non-aqueous electrolytic solutions 5 are the non-aqueous electrolytic solution for a secondary battery mentioned above.

The positive electrode current collector 8 and the negative electrode current collector 3 may be metal foils formed of metals such as aluminum, copper, nickel, and stainless steel.

The positive electrode active material layer 7 includes a positive electrode active material. The positive electrode active material may be a lithium-containing composite oxide or a lithium-containing phosphate compound. Examples of lithium-containing composite oxides include LiMnO₂, LiFeO₂, LiCoO₂, LiMn₂O₄, LiNi₁/₃Co₁/₃Mn₁/₃O₂, LiNi_{0.5}Co_{0.2}Mn_{0.3}O₂, LiNi_{0.6}Co_{0.2}Mn_{0.2}O₂, LiNi_{0.8}Co_{0.1}Mn_{0.1}O₂, and LiNiₓCo_{y}M_{z}O₂ (where 0.01 < x < 1, 0 ≤ y ≤ 1, 0 ≤ z ≤ 1, and x + y + z = 1, and M is at least one element selected from the group consisting of Mn, V, Mg, Mo, Nb, Fe, Cu, and Al), Li_{z}Ni_{(1-x-y)}CoₓM_{y}O₂ (where 0 ≤ x ≤ 0.40, 0 ≤ y ≤ 0.40, and 0.90 ≤ z ≤ 1.20, and M is at least one element selected from the group consisting of Mn, V, Mg, Mo, Nb, and Al), and Li_{z}Co₍₁₋ₓ₎MₓO₂ (where 0 ≤ x ≤ 0.1, and 0.97 ≤ z ≤ 1.20, and M is at least one element selected from the group consisting of Mn, Ni, V, Mg, Mo, Nb, and Al). Examples of lithium-containing phosphate compounds include LiFePO₄, LiCoPO₄, LiMnPO₄, LiNiPO₄, and complexes thereof. When the lithium-containing phosphate compound includes a transition element (Fe, Co, Mn, Ni, or the like), the transition element may be substituted or doped with another element.

The above-mentioned positive electrode active material may be Li_{z}Ni_{(1-x-y)}CoₓM_{y}O₂ (where 0.01 ≤ x ≤ 0.20, 0 ≤ y ≤ 0.30, and 0.90 ≤ z ≤ 1.20, and M is at least one element selected from the group consisting of Mn, V, Mg, Mo, Nb, and Al), Li_{z}Ni_{(1-x-y)}CoₓM_{y}O₂ (where 0.01 ≤ x ≤ 0.15, 0 ≤ y ≤ 0.15, and 0.97 ≤ z ≤ 1.20, and M is at least one element selected from Mn, V, Mg, Mo, Nb, and Al), or Li_{z}Co₍₁₋ₓ₎MₓO₂ (where 0 ≤ x ≤ 0.1, and 0.97 ≤ z ≤ 1.20, and M is at least one element selected from the group consisting of Mn, Ni, V, Mg, Mo, Nb, and Al). In this case, an SEI is easily formed on the electrode surface and it is possible to further improve battery performance as a result.

The negative electrode active material layer 2 includes a negative electrode active material. The negative electrode active material may be, for example, a material capable of absorbing and releasing lithium. Examples of negative electrode active materials include carbon materials such as graphite and amorphous carbon, and oxide materials such as indium oxide, silicon oxide, tin oxide, lithium titanate, zinc oxide, and lithium oxide. The negative electrode active material may be lithium metal or a metal material capable of forming an alloy with lithium. Examples of metals capable of forming an alloy with lithium include Cu, Sn, Si, Co, Mn, Fe, Sb, and Ag. The negative electrode active material may include an alloy including two or three metals including these metals and lithium. These exemplified negative electrode active materials may be used alone or in a combination of two or more.

From the viewpoint of achieving a high energy density, the negative electrode active material may include a carbon material such as graphite and a Si-based active material selected from Si, a Si alloy, a Si oxide or the like. From the viewpoint of achieving both cycle characteristics and a high energy density, the negative electrode active material may include graphite and a Si-based active material. In these cases, the ratio of the mass of the Si-based active material to the total mass of the carbon material and the Si-based active material may be 0.5% by mass or more, 1% by mass or more, or 2% by mass or more and may be 95% by mass or less, 50% by mass or less, or 40% by mass or less.

The positive electrode active material layer 7 and the negative electrode active material layer 2 may further include a binder. Examples of binders include polyvinylideneDifluoride (PVdF), vinylidene fluoride-hexafluoropropylene copolymer, vinylidene fluoride-tetrafluoroethylene copolymer, styrene-butadiene copolymer rubber, carboxymethyl cellulose, polytetrafluoroethylene, polypropylene, polyethylene, polyimide, polyamideimide, polyacrylic acid, polyvinyl alcohol, polyacrylic acid metal salt, acrylic acid-polyacrylonitrile, polyacrylamide, polymethacrylic acid, and copolymers thereof. It is possible for the positive electrode active material layer and the negative electrode active material layer to include the same or different binders.

The positive electrode active material layer 7 and the negative electrode active material layer 2 may further include a conductive auxiliary material for the purpose of reducing resistance. Examples of conductive auxiliary materials include carbonaceous fine particles such as graphite, carbon black, acetylene black, and Ketjenblack, as well as carbon fibers.

The separator 6 may be, for example, a porous film. The porous film may be a film including a resin selected from polyethylene, polypropylene, fluororesin, and the like. The porous film may be a single layer or may have a plurality of layers.

It is possible for the specific form, such as the shape and thickness, of each member forming the power storage device to be appropriately set by a person skilled in the art. The configuration of the power storage device is not limited to the embodiment in Fig. 1 and is able to be appropriately changed.

### Examples

A more detailed description will be given below of the present invention with Examples. The present invention is not limited to the following Examples.

### 1. Non-Aqueous Electrolytic Solution

### (Example 1)

### Synthesis of Compound (1-1) (Compound represented by Formula (1-1))

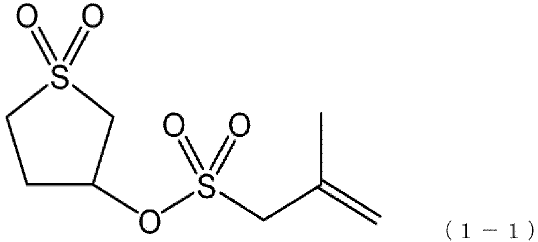

3-sulfolene (236.3 g, 2.0 mol) and 500 mL of water were placed in a 2 L four-neck flask equipped with a stirrer, a condenser, and a thermometer. The reaction solution in the flask was heated to 40°C and 3-sulfolene was dissolved in water until homogeneous. Sodium hydroxide (104.0 g, 2.6 mol) was added to the reaction solution and the reaction solution was stirred for 10 hours while maintaining the temperature at 40°C. Then, the flask was cooled in an ice bath and concentrated sulfuric acid (130.1 g, 1.3 mol) was added dropwise to the reaction solution over 30 minutes. Then, the reaction solution was concentrated to filter the precipitated solid and the obtained filtrate was concentrated to obtain 3-hydroxysulfolane (250.59 g, 92% yield relative to 3-sulfolene).

Sodium 2-methyl-2-propene-1-sulfonate (8.04 g, 50 mmol), N,N-dimethylformamide (0.37 g, 5 mmol), and 30 mL of dichloromethane were placed in a 200 mL four-neck flask equipped with a stirrer, a condenser, a thermometer, and a dropping funnel. Thionyl chloride (7.14 g, 60 mmol) was added dropwise thereto while stirring the reaction solution in the flask at room temperature (25°C). After the dropwise addition was completed, the reaction solution was stirred for 24 hours while maintaining the temperature at 20 to 25°C. Then, water was added to the reaction solution, the mixed liquid was separated, and the oil layer was concentrated to obtain 2-methyl-2-propene-1-sulfonic acid chloride (7.73 g, 100% yield relative to sodium 2-methyl-2-propene-1-sulfonate).

Subsequently, the 3-hydroxysulfolane (5.45 g, 40 mmol) obtained as mentioned above, the 2-methyl-2-propene-1-sulfonic acid chloride (6.80 g, 44 mmol) obtained as mentioned above, and 20 mL of acetonitrile were placed in a 200 mL four-neck flask equipped with a stirrer, a condenser, a thermometer, and a dropping funnel. The flask was cooled in an ice bath and triethylamine (4.05 g, 40 mmol) was added dropwise thereto while stirring the reaction solution in the flask. After the dropwise addition was completed, the reaction solution was stirred for 1 hour while maintaining the temperature at 0 to 5°C. Then, water was added thereto and the precipitated white solid was filtered off. The obtained solid was washed with methanol and then dried under reduced pressure to obtain a compound (1-1) as a white solid (5.84 g, 50% yield relative to 3-hydroxysulfolane).
¹H-NMR (400 MHz, CD₃CN), δ (ppm): 1.93 (m, 3H), 2.53 (m, 2H), 3.18 (m, 2H), 3.36 (dd, 1H), 3.41 (dd, 1H), 3.97 (s, 2H), 5.15 (s, 1H), 5.22 (s, 1H), 5.42 (m, 1H)
¹³C-NMR (100 MHz, CD₃CN) δ (ppm): 22.4, 30.9, 50.0, 57.7, 59.5, 78.0, 121.5, 134.7

### Adjustment of Non-Aqueous Electrolytic Solution

Ethylene carbonate (EC) and ethyl methyl carbonate (EMC) were mixed in a volume ratio of EC:EMC = 30:70 to obtain a mixed non-aqueous solvent. LiPF₆ was dissolved as an electrolyte in the obtained mixed non-aqueous solvent to obtain a LiPF₆ solution with a concentration of 1.0 mol/L. The compound (1-1) was added as an additive for a non-aqueous electrolytic solution to the obtained LiPF₆ solution to prepare a non-aqueous electrolytic solution. The content ratio of the additive for a non-aqueous electrolytic solution (the compound (1-1)) was 1.0% by mass based on the total mass of the non-aqueous electrolytic solution.

### (Example 2)

### Synthesis of Compound (1-5) (Compound represented by Formula (1-5))

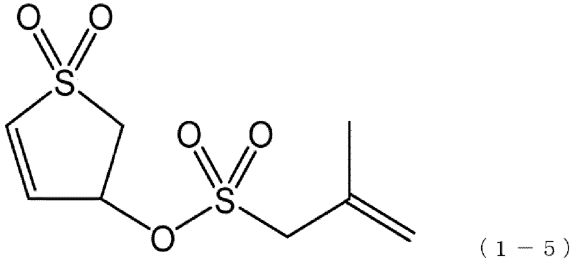

4-hydroxy-2-sulfolene (5.37 g, 40 mmol), 2-methyl-2-propene-1-sulfonic acid chloride (6.80 g, 44 mmol) obtained by the same method as in Example 1, and 20 mL of acetonitrile were placed in a 200 mL four-neck flask equipped with a stirrer, a condenser, a thermometer, and a dropping funnel. The flask was cooled in an ice bath and triethylamine (4.05 g, 40 mmol) was added dropwise thereto while stirring the reaction solution in the flask. After the dropwise addition was completed, the reaction solution was stirred for 1 hour while maintaining the temperature at 0 to 5°C. Then, water was added to the reaction solution and the precipitated white solid was filtered off. The obtained solid was washed with methanol and then dried under reduced pressure to obtain a compound (1-5) as a white solid (6.98 g, 69% yield relative to 4-hydroxy-2-sulfolene).
¹H-NMR (400 MHz, CD₃CN) δ (ppm): 1.93 (m, 3H), 3.37 (dd, 1H), 3.70 (dd, 1H), 4.02 (s, 2H), 5.15 (s, 1H), 5.24 (s, 1H), 5.84 (m1H), 6.84 (d, 1H), 7.04 (d, 1H)
¹³C-NMR (100 MHz, CD₃CN) δ (ppm): 22.4, 54.8, 59.6, 75.9, 121.8, 134.4, 137.0, 137.8

### Preparation of Non-Aqueous Electrolytic Solution

A non-aqueous electrolytic solution was prepared in the same manner as in Example 1 except that the compound (1-5) was used as an additive for a non-aqueous electrolytic solution instead of the compound (1-1).

### (Example 3)

### Synthesis of Compound (1-7) (Compound represented by Formula (1-7))

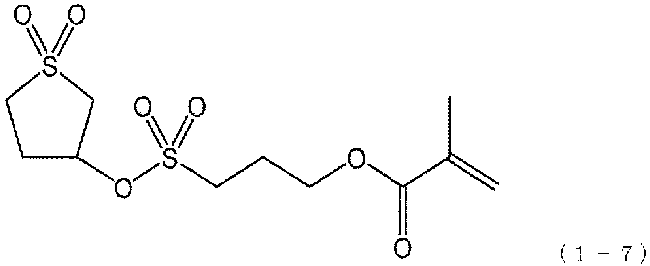

Potassium 3-(methacryloyloxy)propanesulfonate (24.63 g, 100 mmol), N,N-dimethylformamide (2.19 g, 30 mmol), and 60 mL of tetrahydrofuran were placed in a 500 mL four-neck flask equipped with a stirrer, a condenser, a thermometer, and a dropping funnel. Thionyl chloride (65.43 g, 550 mmol) was added dropwise thereto over 30 minutes while stirring the reaction solution in the flask at room temperature. After the dropwise addition was completed, the reaction solution was stirred for 24 hours while maintaining the temperature at 20 to 25°C. Then, water was added to the reaction solution and the mixed liquid was separated. The oil layer was concentrated to obtain 3-(methacryloyloxy)propanesulfonic acid chloride (22.67 g, 100% yield relative to potassium 3-(methacryloyloxy)propanesulfonate).

3-hydroxysulfolane (5.45 g, 40 mmol) obtained by the same method as in Example 1, 3-(methacryloyloxy)propanesulfonic acid chloride (10.86 g, 48 mmol), and 20 mL of acetonitrile were placed in a 200 mL four-neck flask equipped with a stirrer, a condenser, a thermometer, and a dropping funnel. The flask was cooled in an ice bath and triethylamine (4.45 g, 44 mmol) was added dropwise thereto while stirring the reaction solution in the flask. After the dropwise addition was completed, the reaction solution was stirred for 1 hour while maintaining the temperature at 0 to 5°C. Then, water was added to the reaction solution and the precipitated white solid was filtered off. The obtained solid was washed with methanol and then dried under reduced pressure to obtain a compound (1-7) as a white solid (4.02 g, 31% yield relative to 3-hydroxysulfolane).
¹H-NMR (400 MHz, CD₃CN) δ (ppm): 1.93 (m, 3H), 2.15 (m, 2H), 2.54 (m, 2H), 3.17 (m, 2H), 3.33 (t, 2H), 3.37 (ddd, 2H), 4.21 (t, 2H), 5.42 (m, 1H), 5.63 (s, 1H), 6.08 (s, 1H)
¹³C-NMR (100 MHz, CD₃CN) δ (ppm): 18.5, 24.1, 30.9, 48.9, 50.0, 57.7, 63.0, 77.6, 126.3, 137.5, 167.9

### Preparation of Non-Aqueous Electrolytic Solution

A non-aqueous electrolytic solution was prepared in the same manner as in Example 1 except that the compound (1-7) was used as an additive for a non-aqueous electrolytic solution instead of the compound (1-1).

### (Example 4)

### Synthesis of Compound (1-12) (Compound represented by Formula (1-12))

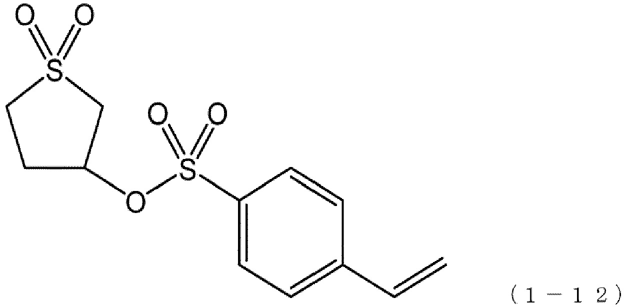

Sodium 4-vinylbenzenesulfonate (24.74 g, 120 mmol) and N,N-dimethylformamide (66.00 g, 90 mmol) were placed in a 500 mL four-neck flask equipped with a stirrer, a condenser, a thermometer, and a dropping funnel. Thionyl chloride (92.79 g, 780 mmol) was added dropwise thereto while stirring the reaction solution in the flask at room temperature. After the dropwise addition was completed, the reaction solution was stirred for 24 hours while maintaining the temperature at 20 to 25°C. Then, water was added to the reaction solution and the mixed liquid was separated. The oil layer was concentrated to obtain 4-vinylbenzenesulfonic acid chloride (18.00 g, 74% yield relative to sodium 4-vinylbenzenesulfonate).

3-hydroxysulfolane (4.77 g, 35 mmol) obtained by the same method as in Example 1, 4-vinylbenzenesulfonic acid chloride (10.86 g, 42 mmol) obtained as mentioned above, and 17.5 mL of acetonitrile were placed in a 200 mL four-neck flask equipped with a stirrer, a condenser, a thermometer, and a dropping funnel. The flask was cooled in an ice bath and triethylamine (4.45 g, 44 mmol) was added dropwise thereto while stirring the reaction solution in the flask. After the dropwise addition was completed, the reaction solution was stirred for 1 hour while maintaining the temperature at 0 to 5°C. Then, water was added thereto and the precipitated white solid was filtered off. The obtained solid was washed with methanol and then dried under reduced pressure to obtain a compound (1-12) as a white solid (5.21 g, 49.2% yield relative to 3-hydroxysulfolane).
¹H-NMR (400 MHz, CD₃CN) δ (ppm): 2.39 (m, 2H), 3.12 (m, 4H), 5.24 (m, 1H), 5.50 (d, 1H), 6.02 (d, 1H), 6.85 (dd, 1H), 7.70 (d, 2H), 7.90 (d, 2H)
¹³C-NMR (100 MHz, CD₃CN) δ (ppm): 30.5, 49.9, 57.3, 78.2, 119.4, 128.3, 129.3, 135.6, 136.1, 144.6

### Preparation of Non-Aqueous Electrolytic Solution

A non-aqueous electrolytic solution was prepared in the same manner as in Example 1 except that the compound (1-12) was used as an additive for a non-aqueous electrolytic solution instead of the compound (1-1).

### (Example 5)

### Synthesis of Compound (1-15) (Compound represented by Formula (1-15))

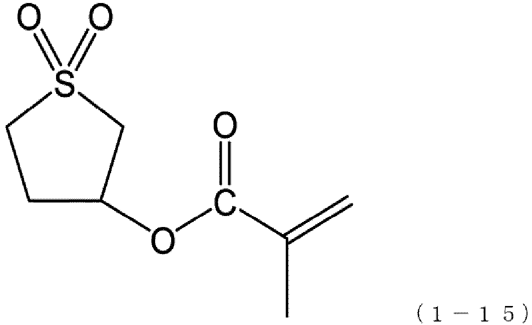

3-hydroxysulfolane (5.45 g, 40 mmol) obtained by the same method as in Example 1, methacryloyl chloride (5.44 g, 52 mmol), and 20 mL of acetonitrile were placed in a 200 mL four-neck flask equipped with a stirrer, a condenser, a thermometer, and a dropping funnel. The flask was cooled in an ice bath and triethylamine (4.86 g, 48 mmol) was added dropwise thereto while stirring the reaction solution in the flask. After the dropwise addition was completed, the reaction solution was stirred for 1 hour while maintaining the temperature at 0 to 5°C. Then, water was added thereto and the precipitated white solid was filtered off. The obtained solid was washed with methanol and then dried under reduced pressure to obtain a compound (1-15) as a white solid (1.96 g, 24.0% yield relative to 3-hydroxysulfolane).
¹H-NMR (400 MHz, CD₃CN) δ (ppm): 1.92 (m, 3H), 2.48 (m, 2H), 3.14 (m, 3H), 3.37 (dd, 1H), 5.51 (m, 1H), 5.68 (s, 1H), 6.11 (s, 1H)
¹³C-NMR (100 MHz, CD₃CN) δ (ppm): 18.3, 29.7, 50.4, 57.4, 71.4, 127.1, 137.2, 167.1

### Preparation of Non-Aqueous Electrolytic Solution

A non-aqueous electrolytic solution was prepared in the same manner as in Example 1 except that the compound (1-15) was used as an additive for a non-aqueous electrolytic solution instead of the compound (1-1).

### (Comparative Example 1)

The LiPF₆ solution (concentration: 1.0 mol/L) before the compound (1-1) was added in Example 1 was used as the non-aqueous electrolytic solution in Comparative Example 1.

### (Comparative Example 2)

A non-aqueous electrolytic solution was prepared in the same manner as in Example 1 except that 1,3-propane sultone (PS, manufactured by Tokyo Chemical Industry Co., Ltd.) was used as the additive for a non-aqueous electrolytic solution instead of the compound (1-1).

### (Comparative Example 3)

### Synthesis of Compound (A) (3-isopropanesulfonyltetrahydrothiophene-1,1-oxide

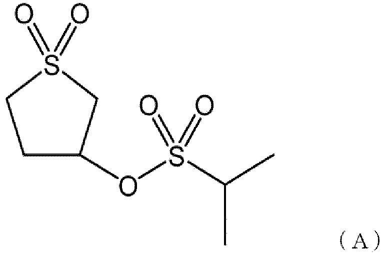

3-hydroxysulfolane (6.81 g, 50 mmol), obtained by the same method as in Example 1, isopropylsulfonic acid chloride (8.56 g, 60 mmol), and 25 mL of acetonitrile were placed in a 200 mL four-neck flask equipped with a stirrer, a condenser, a thermometer, and a dropping funnel. The flask was cooled in an ice bath and triethylamine (5.06 g, 50 mmol) was added dropwise thereto while stirring the reaction solution in the flask. After the dropwise addition was completed, the reaction solution was stirred for 1 hour while maintaining the temperature at 0 to 5°C. Then, water was added thereto and the precipitated solid was filtered off. The obtained solid was washed with methanol and then dried under reduced pressure to obtain a compound (A) (2.31 g, 19% yield relative to 3-hydroxysulfolane).

### Preparation of Non-Aqueous Electrolytic Solution

A non-aqueous electrolytic solution was prepared in the same manner as in Example 1 except that the compound (A) was used as an additive for a non-aqueous electrolytic solution instead of the compound (1-1).

### (Comparative Example 4)

### Synthesis of Compound (B) (3-(4-methylbenzenesulfonyl)tetrahydrothiophene-1,1-dioxide)

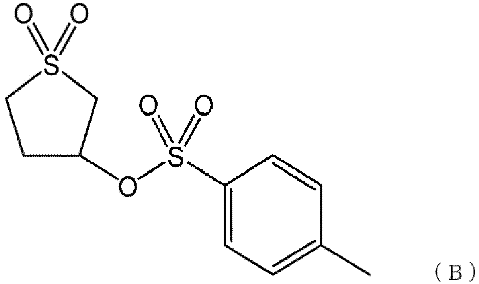

3-hydroxysulfolane (8.17 g, 60 mmol) obtained by the same method as in Example 1, 4-methylbenzenesulfonic acid chloride (13.73 g, 72 mmol), and 30 mL of acetonitrile were placed in a 200 mL four-neck flask equipped with a stirrer, a condenser, a thermometer, and a dropping funnel. The flask was cooled in an ice bath and N,N,N',N'-tetramethylpropanediamine (3.91 g, 30 mmol) was added dropwise thereto while stirring the reaction solution in the flask. After the dropwise addition was completed, the reaction solution was stirred for 1 hour while maintaining the temperature at 0 to 5°C. Then, water was added thereto and the precipitated white solid was filtered off. The obtained white solid was washed with methanol and then dried under reduced pressure to obtain a compound (B) (3.8 4 g, 22% yield relative to 3-hydroxysulfolane).

### Preparation of Non-Aqueous Electrolytic Solution

A non-aqueous electrolytic solution was prepared in the same manner as in Example 1 except that the compound (B) was used instead of the compound (1-1).

### (Comparative Example 5)

### Synthesis of Compound (C) (3-acetoxytetrahydrothiophene-1,1-dioxide)

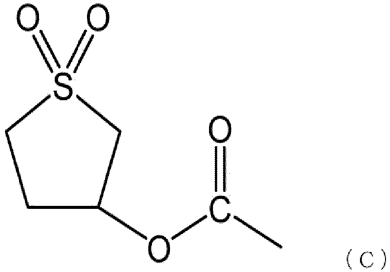

3-hydroxysulfolane (13.62 g, 100 mmol) obtained by the same method as in Example 1, acetic anhydride (11.23 g, 110 mmol), and 10 mL of acetonitrile were placed in a 200 mL four-neck flask equipped with a stirrer, a condenser, a thermometer, and a dropping funnel. The flask was cooled in an ice bath and pyridine (15.82 g, 200 mmol) was added dropwise thereto while stirring the reaction solution in the flask. After the dropwise addition was completed, the reaction solution was stirred for 1 hour while maintaining the temperature at 0 to 5°C. Thereafter, water and dichloromethane were added to the reaction solution and the mixed liquid was separated. The oil layer including dichloromethane was concentrated to obtain a compound (C) (3.97 g, 22% yield relative to 3-hydroxysulfolane).

### Preparation of Non-Aqueous Electrolytic Solution

A non-aqueous electrolytic solution was prepared in the same manner as in Example 1 except that the compound (C) was used as the additive for a non-aqueous electrolytic solution instead of the compound (1-1).

### 2. Preparation of Non-Aqueous Electrolyte Secondary Battery

A positive electrode sheet (manufactured by Hassan Co., Ltd.) including a lithium-containing composite oxide and a negative electrode sheet (manufactured by Hassan Co., Ltd.) including graphite were prepared. The positive electrode sheet had an aluminum foil (thickness 20 µm) as a positive electrode current collector and positive electrode active material layers formed on both surfaces of the aluminum foil. The positive electrode active material layer included a lithium-containing composite oxide (LiNi_{0.5}Co_{0.2}Mn_{0.3}O₂) as a positive electrode active material, carbon black (CB) and carbon (KS) as conductivity-imparting agents, and polyvinylideneDifluoride (PVDF) as a binder. The mass ratio thereof was lithium-containing composite oxide:CB:KS:PVDF = 92:2.5:2.5:3. The negative electrode sheet had a copper foil (thickness 10 µm) as a negative electrode current collector and negative electrode active material layers formed on both surfaces of the copper foil. The negative electrode active material layer included graphite (Gr) as the negative electrode active material and sodium carboxymethylcellulose (CMC) and styrene butadiene rubber (SBR) as binders. The mass ratio thereof was Gr:CMC:SBR = 98:1:1. The negative electrode sheets and positive electrode sheets were alternately stacked via separators formed of propylene to prepare a battery element having a total of 13 layers of electrodes, which were 7 layers of negative electrode sheets and 6 layers of positive electrode sheets.

The prepared battery element was inserted into a bag formed of a laminate film having aluminum (thickness 40 µm) and resin layers covering both surfaces thereof, such that the ends of the positive electrode sheet and the negative electrode sheet protruded from the bag. Each non-aqueous electrolytic solution obtained in the Examples or Comparative Examples was added to the bag and the bag was vacuum sealed to obtain a sheet-shaped non-aqueous electrolyte secondary battery. To increase the adhesion between the electrodes, the sheet-shaped non-aqueous electrolyte secondary battery was sandwiched between glass plates, to which pressure was applied.

### 3. Evaluation

### (Measurement of Initial Capacity)

Each non-aqueous electrolyte secondary battery was charged at 25°C for 1 hour with a current equivalent to 0.1 C and then held at 25°C for 10 hours. Then, each non-aqueous electrolyte secondary battery was charged at a current equivalent to 0.1 C for 5 hours and held at 45°C for 24 hours. Then, at 25°C, discharging was carried out to 3 V with a current equivalent to 0.1 C and then degassing was carried out. After degassing, each non-aqueous electrolyte secondary battery was aged by performing an operation of charging to 4.2 V using a current equivalent to 0.2 C and discharging to 3 V with a current equivalent to 0.2 C, which was repeated for three cycles, an operation of charging to 4.2 V using a current equivalent to 0.5 C and discharging to 3.0 V with a current equivalent to 0.5 C, which was repeated for three cycles, and an operation of charging to 4.2 V with a current equivalent to 1 C and discharging to 3.0 V with a current equivalent to 1 C, which was repeated for three cycles, thereby stabilizing each non-aqueous electrolyte secondary battery. Then, as the initial charging and discharging, charging and discharging were performed with a current equivalent to 2.0 C. The discharge capacity observed in the initial charging and discharging was defined as the "initial capacity".

### (Remaining Capacity Retention Rate and Gas Generation Amount)

The volume of each non-aqueous electrolyte secondary battery after the initial capacity measurement was measured by the Archimedes method and was defined as the "initial volume" of the battery. Each non-aqueous electrolyte secondary battery after the initial charging and discharging was charged to 4.2 V at 1 C at 25°C and then held at 60°C for 30 days. Then, each non-aqueous electrolyte secondary battery was cooled to 25°C and discharged to 3 V with a current equivalent to 1 C. The discharge capacity at this time was defined as the "remaining capacity". The volume of each non-aqueous electrolyte secondary battery after being held at 60°C for 30 days was volume-measured by the Archimedes method and was defined as the "volume after high-temperature storage". The remaining capacity retention rate and gas generation amount after high-temperature storage were calculated by the following formula. The results are shown in Table 3. Remaining capacity retention rate (%) = (remaining capacity/initial capacity) x 100 Gas generation amount (cm3) = volume after high-temperature storage - initial volume

**[Table 3]**

| | Additive for a non-aqueous electrolytic solution | Remaining capacity retention rate (%) | Gas generation amount (cm³) |
|---|---|---|---|
| Ex. 1 | Compound (1-1) | 70 | 0.05 |
| Ex. 2 | Compound (1-5) | 73 | 0.07 |
| Ex. 3 | Compound (1-7) | 71 | 0.17 |
| Ex. 4 | Compound (1-12) | 70 | 0.19 |
| Ex. 5 | Compound (1-15) | 70 | 0.22 |
| Comp. Ex. 1 | None | 63 | 0.59 |
| Comp. Ex. 2 | PS | 66 | 0.44 |
| Comp. Ex. 3 | Compound (A) | 67 | 0.30 |
| Comp. Ex. 4 | Compound (B) | 65 | 0.35 |
| Comp. Ex. 5 | Compound (C) | 66 | 0.36 |

### Reference Signs List

1: lithium-ion battery,
2: negative electrode active material layer,
3: negative electrode current collector,
4: negative electrode,
5: non-aqueous electrolytic solution,
6: separator,
7: positive electrode active material layer,
8: positive electrode current collector,
9: positive electrode.

## Claims

1. An additive for a non-aqueous electrolytic solution comprising:
a cyclic sulfone compound represented by Formula (1): 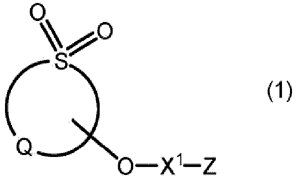 wherein,
Q represents an alkylene group having 4 to 8 carbon atoms which may be substituted or an alkenylene group having 4 to 8 carbon atoms which may be substituted, which forms a cyclic group together with a sulfur atom of a sulfonyl group,
X¹ represents a sulfonyl group or a carbonyl group, and
Z represents a monovalent group represented by Formula (21), (22), (23), or (24): 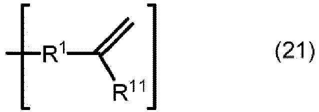 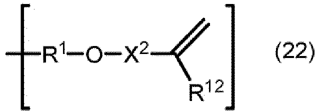 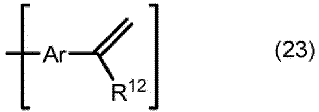 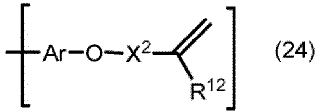 whrerein,
R¹ represents a direct bond or an alkylene group having 1 to 6 carbon atoms which may be substituted,
R¹¹ and R¹² represent a hydrogen atom, a methyl group, or an ethyl group,
when Q is a tetramethylene group and X¹ is a sulfonyl group, R¹¹ is a methyl group or an ethyl group,
X² represents a sulfonyl group or a carbonyl group, and
Ar represents an arylene group which may be substituted.

2. The additive for a non-aqueous electrolytic solution according to claim 1,
wherein X¹ is a sulfonyl group.

3. The additive for a non-aqueous electrolytic solution according to claim 1 or 2,
wherein the cyclic sulfone compound is a compound represented by Formula (11): 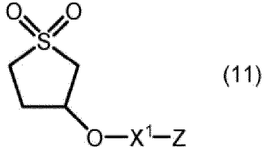 wherein X¹ and Z in Formula (11) are identical to X¹ and Z in Formula (1).

4. The additive for a non-aqueous electrolytic solution according to claim 1 or 2,
wherein the cyclic sulfone compound is a compound represented by Formula (12): 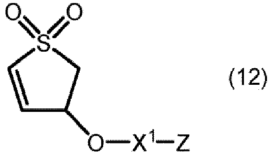 wherein X¹ and Z in Formula (12) are identical to X¹ and Z in Formula (1).

5. The additive for a non-aqueous electrolytic solution according to claim 1 or 2, wherein
R¹ is a direct bond or an alkylene group having 1 to 3 carbon atoms which may be substituted with a fluorine atom, and
Ar is an arylene group which may be substituted with a fluorine atom or a fluoroalkyl group.

6. A non-aqueous electrolytic solution comprising:
the additive for a non-aqueous electrolytic solution according to claim 1 or 2;
a non-aqueous solvent; and
an electrolyte.

7. A power storage device comprising:
the non-aqueous electrolytic solution according to claim 6;
a positive electrode; and
a negative electrode.

8. A lithium-ion battery comprising:
the non-aqueous electrolytic solution according to claim 6;
a positive electrode; and
a negative electrode.

9. A lithium-ion capacitor comprising:
the non-aqueous electrolytic solution according to claim 6;
a positive electrode; and
a negative electrode.

10. A cyclic sulfone compound represented by Formula (11S) or (12S): 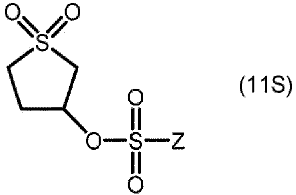 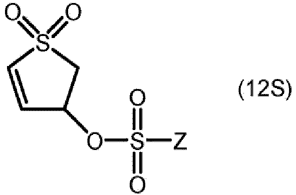 wherein Z represents a monovalent group represented by Formula (21A) or (22A): 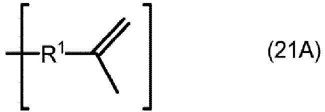 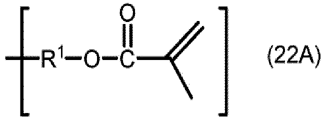 wherein R¹ represents an alkylene group having 1 to 6 carbon atoms which may be substituted with a fluorine atom.

11. The cyclic sulfone compound according to claim 10, represented by the following formula:

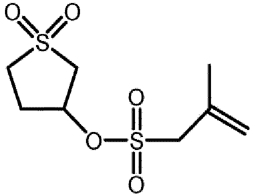

12. The cyclic sulfone compound according to claim 10, represented by the following formula:

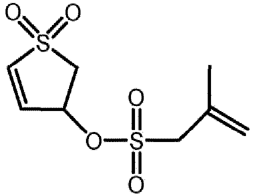

13. The cyclic sulfone compound according to claim 10, represented by the following formula:
